## Europäisches Patentamt
## European Patent Office
## Office européen des brevets

(11) Publication number: **0 075 153**
**B1**

(12) # EUROPEAN PATENT SPECIFICATION

(45) Date of publication of patent specification: **24.06.87**

(51) Int. Cl.⁴: **A 61 B 1/12,** G 01 F 23/30, H 01 H 35/18

(21) Application number: **82108075.1**

(22) Date of filing: **02.09.82**

(54) **Air-liquid supplying device for an endoscope.**

(30) Priority: **17.09.81 JP 146520/81**

(43) Date of publication of application:
**30.03.83 Bulletin 83/13**

(45) Publication of the grant of the patent:
**24.06.87 Bulletin 87/26**

(84) Designated Contracting States:
**AT BE CH DE FR GB IT LI NL SE**

(56) References cited:
**DE-A-2 426 771**
**FR-A-2 021 621**
**FR-A-2 085 345**
**FR-A-2 340 712**
**FR-A-2 475 386**
**GB-A- 315 568**
**US-A-2 780 692**
**US-A-2 907 844**
**US-A-4 176 349**

(73) Proprietor: **OLYMPUS OPTICAL CO., LTD.**
**43-2, 2-chome, Hatagaya Shibuya-ku**
**Tokyo 151 (JP)**

(72) Inventor: **Ueda, Yasuhiro**
**1-22-15, Tokura**
**Kokubunji-shi Tokyo (JP)**

(74) Representative: **Lins, Edgar, Dipl.-Phys. et al**
**Patentanwälte Gramm + Lins Theodor-Heuss-Strasse 2**
**D-3300 Braunschweig (DE)**

Courier Press, Leamington Spa, England.

EP 0 075 153 B1

## Description

This invention relates to an endoscope which comprises an insertion section, a control section, an air-supplying source, and air tube connected to the air-supplying source to guide air through the control section and the insertion section, a vessel for holding a liquid, a liquid tube connected to the vessel to guide liquid through the control section and the insertion section, a changeover control valve provided in the control section of the endoscope and connected to an intermediate part of the air tube and liquid tube, and effecting the selective flow of air and liquid delivered from the air and liquid tube, and an eyepiece lens provided in the control section and arranged at the end of an image guide fiber guiding an image from the distal end of the insertion section into the control section.

Such an endoscope is known from the DE—A—24 26 771. Such an endoscope is generally provided with an air tube through which air is carried from its source into the coeliac cavity of, for example, a patient and a liquid tube through which a liquid is conducted from its vessel into said coeliac cavity. Said air tube and liquid tube are connected to a changeover control valve in the control section of the endoscope. Air or liquid is selectively supplied by operating the changeover control valve. The air source is normally operated during the application of an endoscope. Air from the source is normally released into the atmosphere through the leak hole formed in the changeover control valve. When, therefore, the leak hole is closed with the finger, the air which has been sent forth into the atmosphere up to this point is ejected from the end opening of the air tube. When the changeover control valve is actuated with the leak hole closed, pressure is applied to the liquid vessel by the air from the source. Therefore the liquid held in the vessel flows out through the liquid tube.

When, with the air-liquid supplying device arranged as described above, the liquid supply operation is continued without noticing its absence from the vessel, then air forced into the liquid vessel due to the liquid being pressurized is undesirably ejected into the coeliac cavity of the patient through the liquid tube. In other words, liquid required for the endoscope examination is not supplied, but unnecessary air is brought in. In the extreme case, the difficulties arise that the patient's coeliac cavity is in the danger of being exploded; and it is impossible to carry out the desired observation or the washing of a defective part. Consequently, the liquid supply must always be effected with a close watch kept on the quantity of a liquid still remaining in the vessel.

The conventional process of recognizing the quantity of a liquid held in the vessel comprises applying a transparent vessel and externally observing the quantity of a liquid held therein or confirming its quantity by opening the cap of the vessel. However, the endoscope operator is often concentrated on the observation of the patient's coeliac cavity through the eyepiece section of the endoscope and undesirably tends to forget the confirmation of the quantity of a liquid held in the vessel, erroneously observe said quantity, or try to continuously supply liquid without noticing its absence from the vessel, thereby giving rise to the aforementioned danger of supplying unnecessary air. Moreover, it involves extremely troublesome work to visually confirm the quantity of a liquid in the vessel, each time the endoscope is applied, thereby obstructing the efficient operation of an endoscope.

FR—A—2 475 386 discloses an indication of the actual position of a bendable endoscope head which is controlled by one or two servo motors. The actual position of the servo motors is shown on one or two linear displays which are observed by the operator's eyepiece lens. The information about the actual position of the endoscope head is only relevant during the operation of the endoscope. Therefore, this indication must be seen when viewing to the eyepiece lens.

It is the object of the present invention to avoid the disadvantages with the known endoscopes that the operator must control the content of the liquid vessel in order to prevent air from being pressed through the insertion section when the transport of liquid is intended.

According to the present invention this problem is solved by an endoscope as described above, which comprises:

detection means which detects when the liquid content of the vessel falls below a prescribed level and issues a detection signal denoting the result of said detection; and

an illuminating member for emitting light upon receipt of the detection signal and which is observable through the eye-piece lens.

According to the present invention there is provided a liquid level detector and a light emitting device responding to the liquid level detector and being arranged behind the eyepiece lens so that the indication signal will be seen when looking through the eyepiece lens of the endoscope during the operation so that there is no need for special attention to the change of the liquid level in the vessel, because the fall of the liquid under a predetermined level is unavoidably be noticed by the operator when looking through the eyepiece lens.

This invention can be more fully understood from the following detailed description when taken in conjunction with the accompanying drawings, in which:

Figs. 1 to 3 indicate the arrangement of an endoscope air-liquid supplying device according to a first embodiment of this invention, wherein: Fig. 1 is a schematic sectional view of the whole of the air-liquid supplying device, and Figs. 2 and 3 are fractional sectional views, illustrating the different conditions of said device;

Figs. 4 and 5 are fractional sectional views of an endoscope air-liquid supplying device according to a second embodiment, showing the different conditions of said device;

Figs. 6 and 7 are fractional sectional views of an endoscope air-liquid supplying device according to a third embodiment, indicating the different conditions of said device;

Figs. 8 and 9 are fractional sectional views of an endoscope air-liquid supplying device according to a fourth embodiment, setting forth the different conditions of said device.

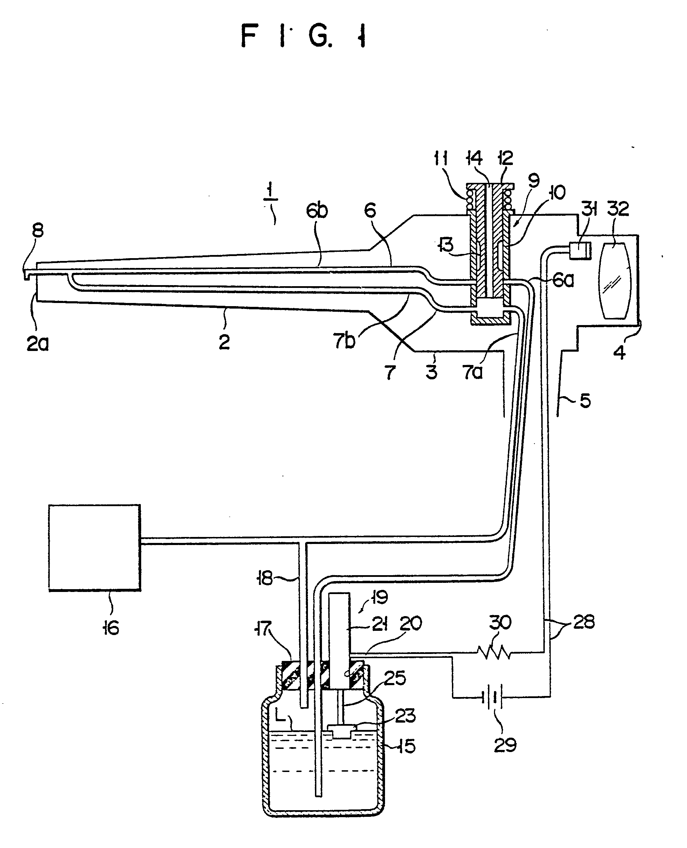

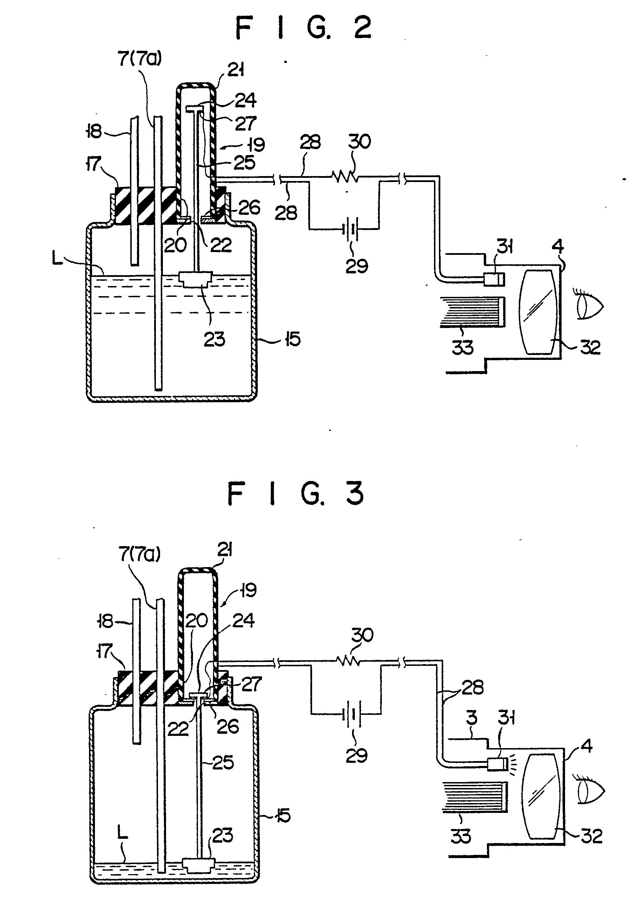

Description is now given with reference to Figs. 1 to 3 of an endoscope air-liquid supplying device according to a first embodiment of this invention. Reference numeral 1 denotes an endoscope constructed by an insertion section 2 and control section 3. The control section 3 is fitted with an eyepiece section 4 and universal cord 5. A liquid tube 6 and air tube 7 extend through the insertion section 2, control section 3 and universal cord 5. The outer end portions of the liquid tube 6 and air tube 7 converge in the distal end portion of the insertion section 2. The converged portions are connected to a nozzle 8 fitted to the end face 2a of the insertion section 2. The liquid tube 6 and air tube 7 are connected to a changeover control valve 9 in the control section 3. The changeover control valve 9 comprises a blind cylinder 10 whose opening faces the outer wall of the control section 3. Inserted into the cylinder 10 is a piston 12 outward urged by a spring 11 set between the cylinder 10 and piston 12. An annular communication hole 13 is formed in the outer peripheral wall of the piston 12. A leak hole 14 axially extends through the piston 12. The upstream side 6a and downstream side 6b of the liquid tube 6 are connected to the peripheral wall of the cylinder 10. The upstream side 6b of the liquid tube 6 are connected to the peripheral wall of the cylinder 10. The upstream side 7a and downstream side 7b of the air tube 7 are connected to those portions of the peripheral wall of the cylinder 10 which are positioned downstream of the liquid tube 6. While the piston 12 is urged by the spring 11, the upstream side 7a and downstream side 7b of the air tube 7 communicate with each other through the lower portion of the cylinder 10, and the upstream side 6a and downstream side 6b of the liquid tube 6 are shut off from each other by the piston 12 (as shown in Fig. 1). When the piston 12 is pressed downward against the urging force of the spring 11, then the upstream side 7a and downstream side 7b of the air tube 7 are shut off from each other by the piston 12. At this time, the upstream side 6a and downstream side 6b of the liquid tube communicate with each other through a communication circular groove 13 formed in the periphery of the piston 12.

The liquid tube 6 and air tube 7 extend out of the universal cord 5. The liquid tube 6 is connected to a vessel 15 holding a liquid L, for example, water. The air tube 7 is connected to an air pump 16. The liquid tube 6 penetrates the cap 17 of the liquid vessel 15 in airtightness. The lower end of the liquid tube 6 is positioned near the bottom of the interior of the liquid vessel 15. A pressurizing tube 18 branched off from the air tube 7 is connected to the cap 17 of the liquid vessel 15. Said cap 17 is provided with detection means 19 for determining the quantity of a liquid L held in the vessel 15. As seen from Figs. 2 and 3, said detection means 19 consists of a cylindrical member 21 whose upper and lower ends are closed and which vertically extends through a fitting hole 20 formed in the cap 17, with the lower end of said cylindrical member 21 inserted into said fitting hole 20 in airtightness. A through hole 22 is formed in the bottom wall of the cylindrical member 21. A rod 25 whose lower end is fitted with a float 23 and whose upper end is provided with a flange 24 is inserted into said through hole 22 in a vertically movable state. A first contact member 26 formed of a metal strip is mounted on the inner wall of the bottom plate of the cylindrical member 21. The underside of the flange 24 is fitted with a second contact member 27 formed of a metal strip and so positioned as to face said first contact member 26. When, as shown in Fig. 2, the float 23 lies on the surface of the liquid L held in the vessel 15, and said liquid L has a prescribed quantity, then the first contact member 26 is widely separated from the second contact member 27. When the liquid L is substantially consumed as shown in Fig. 3, the rod 25 falls with the liquid level, causing the first contact member 26 and second contact member 27 to be electrically connected to each other.

A lead 28 drawn from the first contact member 26 and another lead 28 extending from the second contact member 27 are connected to each other at one end. A power source 29 is provided in the intermediate part of one of the leads 28. A register 30 is connected to the intermediate part of the other lead 28. The leads 28 are inserted into the control section 3 through the universal cord 5 to be connected to a light-emitting diode (LED) 31 acting as display means. When the first contact member 26 and second contact member 27 are brought into contact with each other, the LED 31 emits light rays. The LED 31 is displaced from the optical center of an eyepiece lens 32 provided in the eyepiece section 4. An image guide fiber 33 is provided, which faces at one end an observation window (not shown) formed at the distal end 2a of the insertion section 2 and at the other end is aligned with the optical center of the eyepiece lens 32. Therefore, the endoscope operator can recognize the emission of light rays from the LED 31 while observing the interior of the coeliac cavity through the eyepiece section 4.

Description is now given of the operation of an endoscope air-liquid supplying device embodying this invention. Now let it be assumed that the condition of the coeliac cavity is examined by conducting the insertion section 2 thereinto. In this case, the air pump 16 is previously operated. As a result, air flows from said pump 16 into the cylinder 10 through the upstream side 7a of the air tube 7. If, in this case, the changeover control valve 9 is not actuated as shown in Fig. 1, then air running into the cylinder 10 is released into the atmosphere through the leak hole 14 of the piston 12 which has a smaller resistance than the downstream side 7b of the air tube 7. When it is desired to bring air into the coeliac cavity, it is advised to close the leak hole 14 of the piston 12 with the finger. Then the air which tends to escape through the leak hole 14 is carried into the coeliac cavity through the downstream side 7b of the air tube 7.

When it is desired to supply a liquid, it is advised to press downward the piston 12 with its leak hole 14 closed with the finger and cause the upstream side 7a and downstream side 7b of the air tube 7 to be shut off from each other by the piston 12, and cause the upstream side 6a and downstream side 6b of the liquid tube 6 to communicate with the communication groove 13 of the piston 12. Then the air flowing from the air pump 16 into the air tube 7 runs into the pressurizing tube 18 branched off from the air tube 7, thereby causing pressure to be applied on the surface of the liquid L held in the vessel 15. As a result, the liquid L runs from the upstream side 6a of the liquid tube 6 to the downstream side 6b thereof through the communication hole 13 of the piston 12 and is ejected through the nozzle 8 mounted on the end face 2a of the insertion section 2.

When the liquid is made to run for long hours, the danger sometimes happens that the liquid is all drawn off from the vessel 15, and the air conducted from the pressurizing tube 18 to the liquid tube 6 is brought into the coeliac cavity. When, however, with the endoscope air-liquid supplying device embodying this invention, the liquid L is substantially drawn off from the vessel 15, then the float 23 (Fig. 3) of the detection means 19 is brought downward with the fall of the liquid level. At this time, the second contact member 27 fitted to the underside of the flange 24 is pressed against the first contact member 26 set on the inner bottom wall of the cylindrical member 21, causing the LED 31 to emit a light. Since the illuminating LED 31 is brought into the view of the operator observing the interior condition of the coeliac cavity through the eyepiece section 4, the operator can recognize the substantial absence of the liquid L from the vessel 15 and consequently is prevented from supplying unnecessary air into the coeliac cavity through the air tube 6.

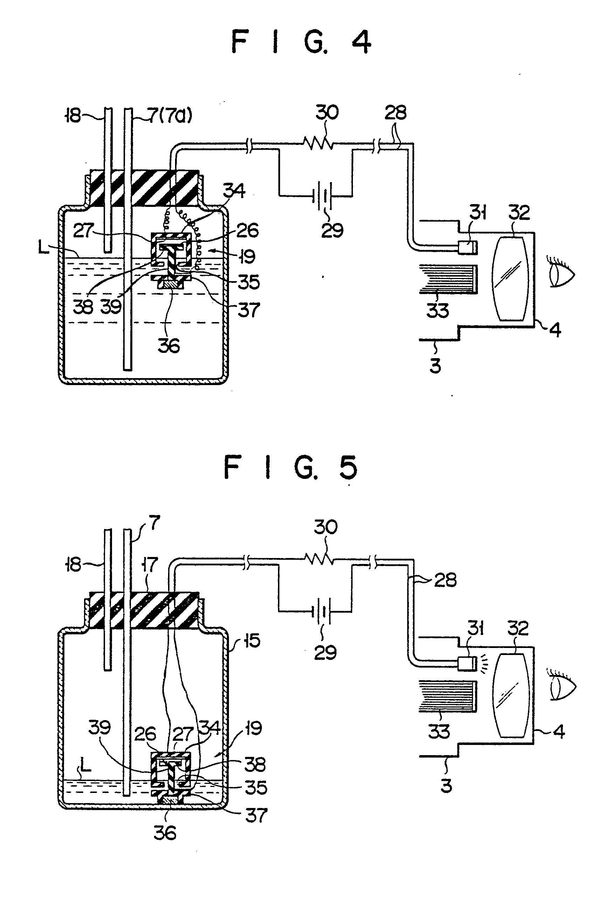

Description is now given with reference to Figs. 4 and 5 of an endoscope air-liquid supplying device according to a second embodiment of this invention. The second embodiment comprises different detection means 19 from the first embodiment. The detection means 19 of the second embodiment comprises a box-shaped member 34 which floats on the liquid L and is open at the bottom and a shaft 39 whose intermediate part vertically moves through a hole drilled in the bottom wall of the box-shaped member 34, whose lower end is fitted with a float 37 carrying a weight 36, and whose upper end is provided with a flange 38. A first contact member 26 is mounted on the surface of the flange 38. A second contact member 27 is fitted to the underside of the upper wall of the box-shaped member 34 in a state facing the first contact member 26. When the vessel 15 is fully filled with the liquid L, then the box-shaped member 34 and the shaft 39 float on the liquid L, with the first and second contact members 26, 27 separated from each other, as shown in Fig. 4. When the liquid L is substantially drawn off from the vessel 15, then the float 37 attaches itself to the inner bottom wall of the vessel 15, and the box-shaped member 34 is brought downward toward the shaft 39. As a result, the first and second contact members 26, 27 are pressed against each other, causing the LED 31 to emit a light. Therefore, the second embodiment can inform the operator of the absence of the liquid L from the vessel 15, as does the first embodiment.

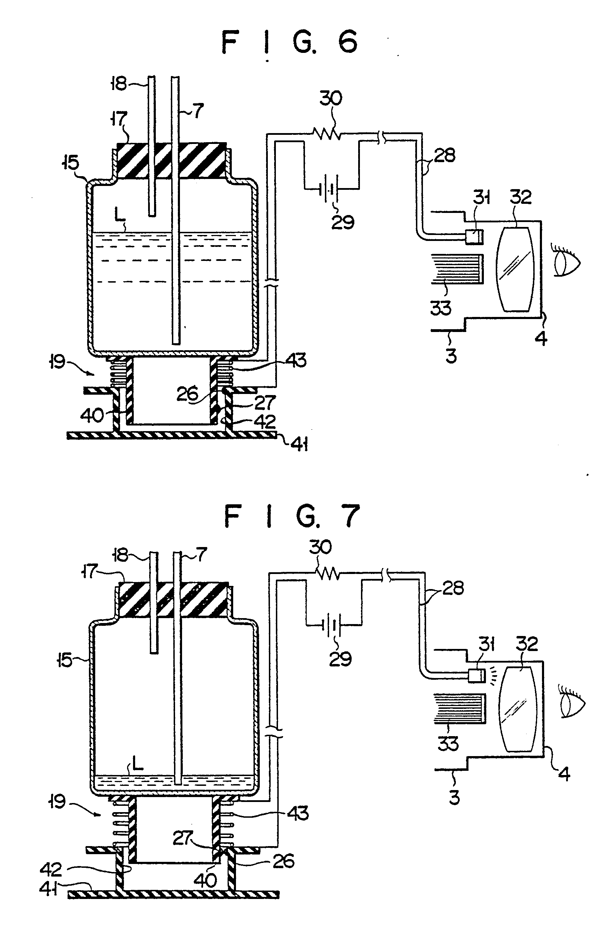

Description is now given with reference to Figs. 6 and 7 of an endoscope air-liquid supplying device according to a third embodiment of this invention. This third embodiment also comprises different detection means 19 from the first embodiment. With the detection means 19 according to the third embodiment, a cylindrical member 40 is fitted to the outer bottom wall of the vessel 15 substantially concentrically therewith. The cylindrical member 40 is inserted into a fitting hole 42 formed in the pedestal 41 in a vertically movable state. A coil spring 43 is provided between the upper plane of the pedestal 41 and the outer bottom wall of the vessel 15 in a state surrounding the cylindrical member 40. The coil spring 43 is compressed by the weight of the liquid L held in the vessel 15. The coil spring 43 progressively lifts the vessel 15 as the liquid L held in the vessel 15 is more reduced. When the liquid L is substantially drawn off from the vessel 15 the coil spring 43 regains its original position, as shown in Fig. 7 and lifts the vessel 15. A first contact member 26 is set in the proximity of the upper portion of the inner peripheral wall of the fitting hole 42. A second contact member 27 is provided in the proximity of the lower portion of the outer peripheral wall of the cylindrical member 40 in a state facing the first contact member 26. When the vessel 15 is fully filled with the liquid L, that is, compresses the coil spring 43, then the first and second contact members 26, 27 are separated from each other. Conversely when the liquid L is substantially drawn off from the vessel 15, which in turn is lifted by the urging force of the coil spring 43, then the second contact member 27 fitted to the outer peripheral wall of the cylindrical member 40 which is now lifted with the vessel 15 is pressed against the first contact member 26. As a result, the LED 31 emits a light, thereby enabling the operator to recognize the substantial absence of the liquid L from its indicated current weight.

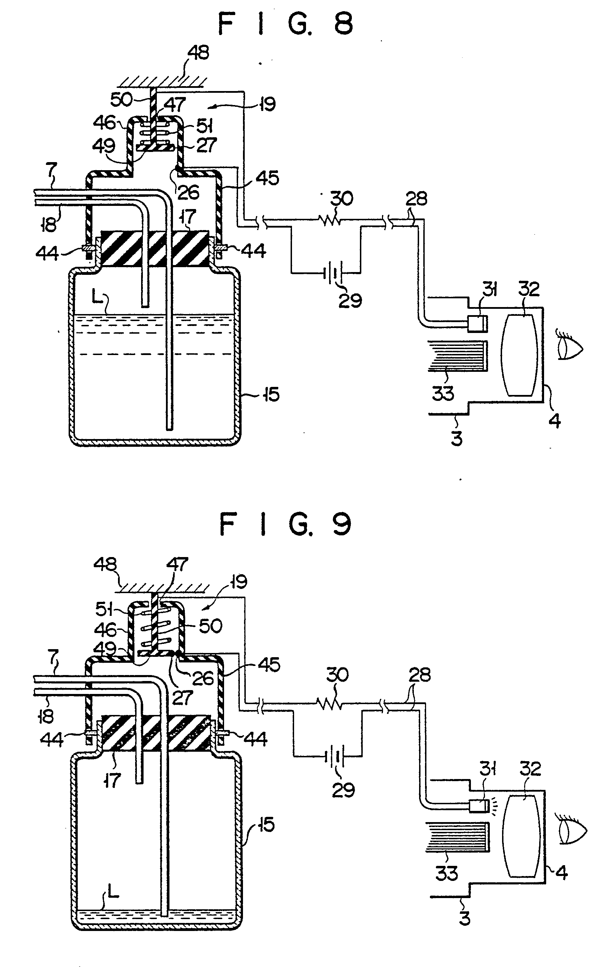

Description is now given with reference to Figs. 8 and 9 of an endoscope air-liquid supplying device according to a fourth embodiment of this invention. The detection means 19 of this fourth embodiment detects the level of the liquid L from changes in its weight as in the third embodiment. The detection means 19 comprises a pair of pins 44 provided on the upper peripheral wall of the vessel 15 in a state projecting outward in the opposite directions. These pins 44 are respectively inserted into holes drilled in the lower part of the suspended member 45 which in turn is fixed in place with its both end portions engaged with the pins 44. An upward extending hollow projection 46 is provided in the upper portion of the suspended member 45. A first contact member 26 is provided on the bottom of the inner wall of one side of said suspended member 45. A penetrating hole 47 is drilled in the center of the rop of the projection 46. Inserted through said hole 47 is a shaft 50 whose upper end is secured to the fitting member 48 and whose lower end is fitted with a flange 49. The intermediate part of said shaft 50 can vertically move through said hole 47. A compression coil spring 51 is provided between the upper surface of the flange 49 and the underside of the upper portion of the upward extending projection 46. A second contact member 27 is provided on the outer peripheral wall of the flange 49. When the vessel 15 is fully filled with the liquid L, the compression coil spring 51 is compressed as shown in Fig. 8 by the weight of the liquid L. Conversely when the liquid L is substantially drawn off from the vessel 15, the compression coil spring regains its original position as shown in Fig. 9. At this time the suspended member 45 is lifted with the vessel 15 by the righting moment of the compression coil spring 51. As a result, the first contact member 26 mounted on the inner wall of the upward extending projection 46 of the suspended member 45 is pressed against the second contact member 27 formed on the outer peripheral wall of the flange 49, causing the LED 31 to emit a light. Therefore, this fourth embodiment enables the operator to recognize the substantial absence of the liquid L from its indicated current weight, as does the third embodiment.

With the aforementioned endoscope air-liquid supplying device of this invention which selectively supplies air or liquid by the operation of the changeover control valve, detection means detects the amount of a liquid held in a vessel. Upon receipt of an output signal from the detection means, the display means indicates the absence of the liquid from the vessel, thereby enabling the operator to unfailingly recognize the withdrawal of the liquid from the vessel. Consequently, the endoscope air-liquid supplying device of this invention has the advantages that it is possible to eliminate the troublesome work of visually recognizing the liquid content of a vessel as has been practised in the conventional air-liquid supplying device, and consequently elevate the efficiency of operating an endoscope; and the danger is avoided of continuing liquid supply without noticing the absence of a liquid and undesirably introducing unnecessary air into the coeliac cavity of a patient through a liquid tube.

**Claims**

1. An endoscope which comprises:
— an insertion section (2);
— a control section (3);
— an air-supplying source (16);
— an air tube (7) connected to the air-supplying source (16) to guide air through the control section (3) and the insertion section (2);
— a vessel (15) for holding a liquid;
— a liquid tube (6) connected to the vessel (15) to guide liquid through the control section (3) and the insertion section (2);
— a changeover control valve (9) provided in the control section (3) of the endoscope (1) and connected to an intermediate part of the air tube (7) and liquid tube (6), and effecting the selective flow of air and liquid delivered from the air and liquid tube; and
— an eyepiece lens (32) provided in the control section (3) and arranged at the end of an image guide fiber (33) guiding an image from the distal end of the insertion section (2) into the control section (3),
characterized by comprising:
— detection means (19) which detects when the liquid content of the vessel (15) falls below a prescribed level and issues a detection signal denoting the result of said detection; and
— an illuminating member (31) for emitting light upon receipt of the detection signal and which is observable through the eyepiece lens (32).

2. The endoscope according to claim 1, characterized in that the detection means (19) comprises:
— a float (23) kept buoyant by a liquid held in the vessel;
— a movable member (25) which projects from the liquid level and rises or falls with the vertical shifting of the liquid level;
— a stationary member (21) which is fixed to the vessel and allows for the insertion of the movable member in a vertically movable state; and
— first and second contact members (26, 27) which are respectively fitted to said movable and stationary members and brought into electric contact when the movable member descends due to the fall of the liquid content of the vessel from a prescribed level.

3. The endoscope according to claim 2, characterized in that the vessel (15) comprises an opening formed in the upper portion and a cap (17) for closing said opening; the fixed member (21) comprises a cylindrical member which vertically extends through the cap and whose lower end is provided with a closed wall; the movable member (25) comprises a shaft which vertically projects from the float, penetrates the closed wall, extends through the cylindrical member in a vertically movable state, and is provided with a flange (24) at the projecting end; and the first and second contact members are respectively provided inside of the closed wall and on the underside of the flange in a mutually facing state.

4. The endoscope according to claim 1, characterized in that the detection means (19) comprises:

— first and second movable members (34, 39) which are kept buoyant by the liquid held in the vessel, project from the liquid level, and vertically move with the rise and fall of the liquid level, said first movable member being so arranged as to be vertically moved relative to the second movable member; and

— first and second contact strips (26, 27) which are respectively formed on the first and second movable members and electrically contact each other, when the fall of the liquid content of the vessel from a prescribed level causes the downward movement of the first movable member to be obstructed by the bottom wall of the vessel, and the second movable member is moved downward relative to the first movable member.

5. The endoscope according to claim 4, characterized in that the second movable member (34) comprises a box-shaped member whose upper wall lies above the liquid level; the first movable member (39) comprises a shaft which vertically extends from the liquid level, and whose extended end is positioned in the box-shaped member and provided with a flange (38); and the first and second contact members are respectively mounted on the surface of the flange of the shaft and on the underside of the upper wall of the box-shaped member in a mutually facing state.

6. The endoscope according to claim 1, characterized in that the detection means (19) comprises a fixed member (41) and a spring (43) for elastically holding the vessel (15) so as to let it vertically move between the first and second positions in accordance with the weight of a liquid held in the vessel; the first and second contact members (26, 27) are respectively fitted to the vessel and fixed member in a mutually facing state; and when the vessel assumes the second position due to the substantial withdrawal of a liquid from the vessel the first and second contact members are electrically connected together.

**Patentansprüche**

1. Endoskop mit
— einem Einführungsteil (2),
— einem Steuerteil (3),
— einer Luftzuführung (16),
— einer mit der Luftzuführung (16) verbundenen Luftleitung (7) zur Leitung von Luft durch das Steuerteil (3) und das Einführungsteil (2),
— einem Behälter (15) für eine Flüssigkeit,
— einer mit dem Behälter (15) verbundenen Flüssigkeitsleitung (6) zur Leitung von Flüssigkeit durch das Steuerteil (2) und das Einführungsteil (3),
— einem Umschalt-Steuerventil (9), das im Steuerteil (3) des Endoskops (1) angeordnet und mit einem Zwischenabschnitt der Luftleitung (7) und der Flüssigkeitsleitung (6) verbunden ist und eine aus der Luftleitung und der Flüssigkeitsleitung kommende selektive Strömung von Luft und Flüssigkeit bewirkt und
— einer Okularlinse (32), die in dem Steuerteil (3) vorgesehen und am Ende einer Bildleiterfaser (33) angeordnet ist, die ein Bild vom distalen Ende des Einführungsteils (2) zum Steuerteil (3) leitet.
gekennzeichnet durch
— einen Detektor (19), der detektiert, wann der Flüssigkeitsspiegel in dem Behälter (15) unter einen vorgeschriebenen Pegel sinkt und ein das Resultat der Detektion kennzeichnendes Detektionssignal abgibt und
— eine Beleuchtungsvorrichtung (31) zur Aussendung von Licht beim Empfang des Detektionssignals, die durch die Okularlinse (32) beobachtbar ist.

2. Endoskop nach Anspruch 1, gekennzeichnet durch folgende Teile des Detektors (19):
— einen Schwimmer (23), der auf der Flüssigkeit in dem Behälter schwimmt,
— ein bewegbares Teil (25), das sich von dem Flüssigkeitspegel aus erstreckt und mit der vertikalen Verschiebung des Flüssigkeitspegels steigt oder fällt,
— ein ortsfestes Teil (21), das an dem Behälter befestigt ist und die Einführung des bewegbaren Teils in vertikal bewegbarer Weise erlaubt und
— durch erste und zweite Kontaktteile (26, 27), die am bewegbaren bzw. ortsfesten Teil angebracht sind und in elektrischen Kontakt zueinander kommen, wenn das bewegbare Teil aufgrund des Abfalls des Flüssigkeitspegels in dem Behälter unter einen vorgeschriebenen Pegel sinkt.

3. Endoskop nach Anspruch 2, dadurch gekennzeichnet, daß der Behälter (15) in seinem oberen Teil eine Öffnung und eine Kappe (17) zum Verschließen der Öffnung aufweist, daß das ortsfeste Teil (21) ein zylidrisches Glied aufweist, das sich vertikal durch die Kappe (17) erstreckt und dessen unteres Ende mit einer geschlossenen Wand versehen ist,
daß das bewegbare Teil einen Schacht aufweist, der vertikal von dem Schwimmer hervorragt, durch die geschlossene Wand läuft, sich in vertikal bewegbarer Weise durch das zylindrische Teil erstreckt und einen Flansch (24) an seinem hochstehenden Ende aufweist und daß die ersten und zweiten Kontaktteile einander gegenüberliegend an der Innenseite der geschlossenen Wand bzw. der Unterseite des Flansches angeordnet sind.

4. Endoskop nach Anspruch 1, gekennzeichnet durch folgende Merkmale des Detektors (19):
— ein erstes und ein zweites bewgbares Teil (34, 39), die auf der Flüssigkeit in dem Behälter schwimmen, aus der Flüssigkeitsoberfläche herausragen und sich mit dem Anstieg und dem Abfall des Flüssigkeitspegels vertikal bewegen, wobei das erste bewegbare Teil relativ zum zweiten bewegbaren Teil vertikal bewegbar angeordnet ist und
— durch erste und zweite Kontaktstreifen (26, 27), die am ersten bzw. zweiten bewegbaren Teil angeordnet sind und miteinander einen elektrischen Kontakt geben, wenn der Abfall der Flüssigkeit in dem Behälter unter einen vorbestimmten Pegel bewirkt, daß die Abwärtsbewegung des ersten bewegbaren Teils durch den Boden des Behälters gestört wird und daß das zweite bewegbare Teil relativ zum ersten bewegbaren Teil nach unten bewegt wird.

5. Endoskop nach Anspruch 4, dadurch gekennzeichnet, daß das zweite bewegbare Teil (34) ein kastenförmiges Teil ist, dessen obere Wand oberhalb des Flüssigkeitspegels liegt, daß das erste bewegbare Teil (39) einen Schaft aufweist, der vertikal von dem Flüssigkeitspegel nach oben ragt und dessen oberes Ende mit einem Flansch (38) versehen und in dem kastenförmigen Teil untergebracht ist, und daß die ersten und zweiten Kontaktteile einander gegenüberliegend auf der Oberfläche des Flansches des Schafts bzw. auf der Unterseite der Oberwand des kastenförmigen Teils befestigt sind.

6. Endoskop nach Anspruch 1, dadurch gekennzeichnet, daß der Detektor (19) ein ortsfestes Teil (41) und eine Feder (43) aufweist, die den Behälter (15) elastisch hält, so daß er sich vertikal zwischen einer ersten und einer zweiten Position in Abhängigkeit von dem Gewicht der in dem Behälter befindlichen Flüssigkeit bewegt, daß die ersten und zweiten Kontaktteile (26, 27) einander gegenüberliegend an dem Behälter und an dem ortsfesten Teil befestigt sind und elektrisch miteinander verbunden werden, wenn der Behälter aufgrund eines wesentlichen Absaugens der Flüssigkeit aus dem Behälter die zweite Position einnimmt.

**Revendications**

1. Endoscope qui comprend:
— une section d'introduction (2),
— une section de commande (3),
— une source d'alimentation d'air (16),
— un tube d'air (7) raccordé à la source d'alimentation d'air (16) pour guider l'air à travers la section de commande (3) et la section d'introduction (2),
— un récipient (15) destiné à contenir un liquide,
— un tube de liquide (6) raccordé au récipient (15) pour guider le liquide à travers la section de commande (3) et la section d'insertion (2),
— une valve de commande de commutation (9) prévue dans la section de commande (3) de l'endoscope (1), raccordée à une partie intermédiaire du tube d'air (7) et du tube de liquide (6) et déterminant l'écoulement sélectif de l'air et du liquide provenant du tube d'air et du tube de liquide, et
— une lentille d'oculaire (32) prévue dans la section de commande (3) et disposée à l'extrémité d'une fibre de guidage d'image (33) qui guide une image depuis l'extrémité distale de la section d'introduction (2) dans la section de commande (3),
caractérisé en ce qu'il comprend:
— un dispositif de détection (19) qui détecte le moment où le volume de liquide contenu dans le récipient (15) descend en dessous d'un niveau prescrit et émet un signal de détection indiquant le résultat de la détection, et
— un élément d'éclairage (31) pour émettre de la lumière à la réception du signal de détection et qui peut être observé à travers la lentille d'oculaire (32).

2. Endoscope suivant la revendication 1, caractérisé en ce que le dispositif de détection (19) comprend:
— un flotteur (23) maintenu flottant par un liquide contenu dans le récipient,
— un élément mobile (25) qui s'étend depuis le niveau du liquide et qui monte ou descend à mesure que le niveau du liquide se déplace verticalement,
— un élément fixe (21) qui est fixé au récipient et permet l'introduction de l'élément mobile dans un état verticalement mobile, et
— un premier et un second organe de contact (26, 27) qui sont respectivement prévus sur l'élément mobile et l'élément fixe et qui sont amenés en contact électrique lorsque l'élément mobile descend par suite d'une diminution du volume de liquide contenu dans le récipient à partir d'un niveau prescrit.

3. Endoscope suivant la revendication 2, caractérisé en ce que le récipient (15) comprend une ouverture formée dans sa partie supérieure et un bouchon (17) pour fermer cette ouverture, l'élément fixe (21) comprend un élément cylindrique qui traverse verticalement le bouchon et dont l'extrémité inférieure est pourvue d'une paroi close, l'élément mobile (25) comprend une tige qui s'étend verticalement à partir du flotteur, pénètre dans la paroi close, traverse l'élément cylindrique dans un état verticalement mobile et est pourvue d'une collerette (24) à son extrémité opposée au flotteur, et le premier ainsi que le second organe de contact sont respectivement prévus à l'intérieur de la paroi close et sur la face inférieure de la collerette de manière à se faire face.

4. Endoscope suivant la revendication 1, caractérisé en ce que le dispositif de détection (19) comprend:
— un premier et un second élément mobile (34, 39) qui sont maintenus flottants par le liquide contenu dans le récipient, qui s'étendent à partir du niveau du liquide et qui se déplacent verticalement à mesure que le niveau du liquide monte et descend, le premier élément mobile étant agencé de manière à être déplacé verticalement par rapport au second élément mobile, et
— une première et une seconde bande de contact (26, 27) qui sont respectivement formées sur le premier et le second élément mobile et qui entrent en contact électrique l'une avec l'autre lorsque le diminution du volume de liquide contenu dans le récipient à partir d'un niveau prescrit provoque l'arrêt de la descente du premier élément mobile par la paroi de fond du récipient, et que le second élément mobile est déplacé vers le bas par rapport au premier élément mobile.

5. Endoscope suivant la revendication 4, caractérisé en ce que le second élément mobile (34) comprend un élément en forme de cube dont la paroi supérieure est disposée au-dessus du niveau du liquide, le premier élément mobile (39) comprend une tige qui s'étend verticalement à partir du niveau du liquide et dont l'extrémité libre est place dans l'élément en forme de cube et est pourvue d'une collerette (38), et le premier ainsi que le second organe de contact sont montés respectivement sur la surface de la collerette de la tige et sur le dessous de la paroi supérieure de l'élément en forme de cube de manière à se faire face.

6. Endoscope suivant la revendication 1, caractérisé en ce que le dispositif de détection (19) comprend un élément fixe (41) et un ressort (43) pour retenir élastiquement le récipient (15) de manière à lui permettre de se déplacer verticalement entre une première et une seconde position en fonction du poids d'un liquide y contenu, le premier et le second organe de contact (26, 27) sont montés respectivement sur le récipient et sur l'élément fixe de manière à se faire face et, lorsque le récipient occupe sa seconde position par suite du soutirage substantiel d'un liquide de ce récipient, le premier et le second organe de contact sont connectés électriquement l'un à l'autre.

0 075 153

# F I G. 1

# F I G. 2

# F I G. 3

# F I G. 4

# F I G. 5

# F I G. 6

# F I G. 7

F I G. 8

F I G. 9